(19) Europäisches Patentamt
European Patent Office
Office européen des brevets

(11) Veröffentlichungsnummer : **0 103 798**
**B1**

(12) **EUROPÄISCHE PATENTSCHRIFT**

(45) Veröffentlichungstag der Patentschrift : **30.12.86**

(21) Anmeldenummer : **83108717.6**

(22) Anmeldetag : **05.09.83**

(51) Int. Cl.[4] : **C 07 D249/08**, C 07 D233/60, A 01 N 43/64, A 01 N 43/50

(54) **Substituierte Azolylalkyl-t-butyl-ketone und -carbinole.**

(30) Priorität : **18.09.82 DE 3234627**

(43) Veröffentlichungstag der Anmeldung : **28.03.84 Patentblatt 84/13**

(45) Bekanntmachung des Hinweises auf die Patenterteilung : **30.12.86 Patentblatt 86/52**

(84) Benannte Vertragsstaaten : **AT BE CH DE FR GB IT LI NL SE**

(56) Entgegenhaltungen :
**EP-A- 0 016 323**
**EP-A- 0 031 911**
**EP-A- 0 032 200**
***Die Akte enthält technische Angaben, die nach dem Eingang der Anmeldung eingereicht wurden und die nicht in dieser Patentschrift enthalten sind.***

(73) Patentinhaber : **BAYER AG**
**Konzernverwaltung RP Patentabteilung**
**D-5090 Leverkusen 1 Bayerwerk (DE)**

(72) Erfinder : **Reiser, Wolf, Dr.**
**Kiebitzweg 12a**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Brandes, Wilhelm, Dr.**
**Eichendorfstrasse 3**
**D-5653 Leichlingen (DE)**
Erfinder : **Reinecke, Paul, Dr.**
**Lessingstrasse 11**
**D-5090 Leverkusen 3 (DE)**

**EP 0 103 798 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte Azolylalkyl-t-butyl-ketone und -carbinole, ein Verfahren zu ihrer Herstellung und ihre Verwendung als Pflanzenschutzmittel und als Zwischenprodukte für die Synthese von weiteren Pflanzenschutzmitteln.

Es ist bereits bekannt geworden, daß bestimmte substituierte Azolylalkyl-t-butyl-ketone und -carbinole wie z. B. 1-(2,4-Dichlorphenoxy)-3,3-dimethyl-1-(1,2,4-triazol-1-yl)-butan-2-on oder 4,4-Bis-(chlormethyl)-1-phenyl-1-(1,2,4-triazol-1-yl)-pentan-3-on oder 2,2-Dimethyl-1-fluor-5-phenyl-4-(1,2,4-triazol-1-yl)-pentan-3-ol oder 5-(3,4-Dichlorphenyl)-2,2-dimethyl-1-fluor-4-(1,2,4-triazol-1-yl)-pentan-3-ol fungizide Wirksamkeit besitzen (vgl. DE-A 2 407 143, DE-A 2 951 164 bzw. EP-A 0 031 911 und DE-A 2 951 163 bzw. EP-A 0 032 200).

Die Wirkung dieser Azolderivate ist jedoch in bestimmten Indikationsbereichen, insbesondere bei niedrigen Aufwandmengen und -konzentrationen nicht immer ganz zufriedenstellend.

Im übrigen sind aus der EP-A 0 016 323 Allyltriazol-Derivate mit pflanzenwachstumsregulierender und fungizider Wirkung bekannt. Der in der Seitenkette gegebenenfalls vorhandene Cycloalkyl- oder Cycloalkenyl-Rest ist jeweils über Alkenyl mit dem zentralen Kohlenstoffatom verbunden. Entsprechende Stoffe mit gesättigter Seitenkette werden jedoch nicht beschrieben.

Es wurden nun neue substituierte Azolylalkyl-t-butyl-ketone und -carbinole der allgemeinen Formel (I)

$$R-\underset{\substack{|\\ Az}}{CH}-A-C(CH_3)_3 \qquad (I)$$

in welcher

Az für Imidazol-1-yl oder 1,2,4-Triazol-1-yl steht,

A für die Carbonylgruppe oder die Hydroxymethylengruppe steht und

R für gegebenenfalls ein- bis fünffach durch Halogen, Alkyl mit bis zu 4 Kohlenstoffatomen und/oder Alkoxy mit bis zu 4 Kohlenstoffatomen gleich oder verschieden substituiertes Cycloalkylalkyl und Cycloalkenylalkyl mit jeweils 3 bis 9 Kohlenstoffatomen im Cycloalkyl- bzw. Cycloalkenylteil und bis zu 4 Kohlenstoffatomen im jeweiligen Alkyl steht,

sowie deren pflanzenverträgliche Säureadditionssalze und Metallsalzkomplexe gefunden.

Weiterhin wurde gefunden, daß man die substituierten Azolylalkyl-t-butyl-ketone der allgemeinen Formel (I) sowie deren pflanzenverträgliche Säureadditionssalze und Metallsalzkomplexe erhält, wenn man Azolylmethyl-t-butyl-ketone der Formel (II)

$$Az-CH_2-\underset{\substack{\|\\ O}}{C}-C(CH_3)_3 \qquad (II)$$

in welcher Az die oben angegebene Bedeutung hat, mit einem Alkylierungsmittel der allgemeinen Formel (III)

$$R—Z \qquad (III)$$

in welcher

R die oben angegebene Bedeutung hat und

Z für eine elektronenanziehende Abgangsgruppierung steht,

in Gegenwart einer Base und in Gegenwart eines organischen Verdünnungsmittels, oder in einem wässrigorganischen Zweiphasensystem in Gegenwart eines Phasentransferkatalysators, umsetzt und gegebenenfalls die dabei erhaltenen Keto-Verbindungen nach bekannten Methoden zu den entsprechenden Carbinolen reduziert, und gegebenenfalls an die erhaltenen Verbindungen der allgemeinen Formel (I) eine pflanzenverträgliche Säure oder ein pflanzenverträgliches Metallsalz addiert.

Die neuen Azolylalkyl-t-butyl-ketone und -carbinole der allgemeinen Formel (I) weisen starke fungizide Eigenschaften auf und sind daher als Pflanzenschutzmittel anwendbar.

Ueberraschenderweise zeigen die erfindungsgemäßen substituierten Azolylalkyl-t-butyl-ketone und -carbinole ein bessere fungizide Wirksamkeit als die aus dem Stand der Technik bekannten Azole 1-(2,4-Dichlorphenoxy)-3,3-dimethyl-1-(1,2,4-triazol-1-yl)-butan-2-on oder 4,4-Bis-(chlormethyl)-1-phenyl-1-(1,2,4-triazol-1-yl)-pentan-3-on oder 2,2-Dimethyl-1-fluor-5-phenyl-4-(1,2,4-triazol-1-yl)-pentan-3-ol oder 5-(3,4-Dichlorphenyl)-2,2-dimethyl-1-fluor-4-(1,2,4-triazol-1-yl)-pentan-3-ol, welche chemisch und wirkungsmäßig naheliegende Verbindungen sind.

Außerdem sind die erfindungsgemäßen substituierten Azolylalkyl-t-butyl-ketone und -carbinole der allgemeinen Formel (I) interessante Zwischenprodukte zur Herstellung von weiteren Pflanzenschutzwirkstoffen. So können zum Beispiel nach bekannten Verfahren erfindungsgemäße Keto-Verbindungen mit

Hydroxylamin bzw. dessen Derivaten oder mit Hydrazinen in die entsprechenden Oxime und Hydrazone überführt werden. Weiterhin können erfindungsgemäße Carbinole mit Alkylierungs- oder Acylierungsmitteln in die entsprechenden Ether und Ester überführt werden.

Die erfindungsgemäßen Stoffe stellen somit eine wertvolle Bereicherung des Standes der Technik dar.

Die erfindungsgemäßen substituierten Azolylalkyl-t-butyl-ketone und -carbinole sind durch die allgemeine Formel (I) allgemein definiert.

Bevorzugt sind Verbindungen der allgemeinen Formel (I), in welcher

Az und A die in der Erfindungsdefinition angegebenene Bedeutung haben und

R für gegebenenfalls ein- bis dreifach, gleich oder verschieden durch Methyl, Methoxy, Ethyl, oder Chlor substituiertes Cyclopropylmethyl, Cyclobutylmethyl, Cyclopentylmethyl, Cyclohexylmethyl, Cyclohexenylmethyl, Cyclohexadienylmethyl, Cycloheptylmethyl oder Cycloheptenylmethyl steht.

Im einzelnen seien außer den bei den Herstellungsbeispielen genannten Verbindungen die folgenden Verbindungen der allgemeinen Formel (I) genannt :

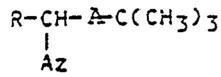

| R | Az | A |
|---|---|---|
| 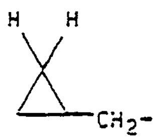 | 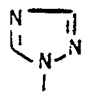 | 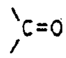 |
| 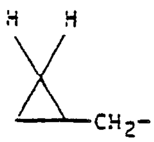 | 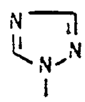 | 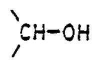 |
| 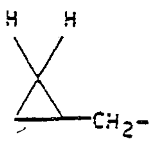 | 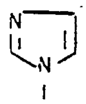 | 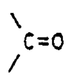 |
| 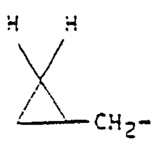 | 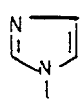 | 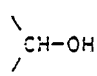 |
| 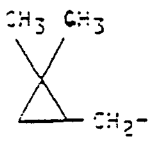 | 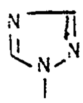 | 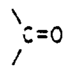 |
| 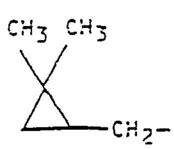 | 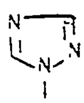 | 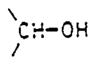 |

(Fortsetzung)

| R | Az | A |
| --- | --- | --- |
| 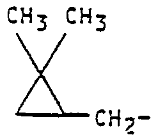 | 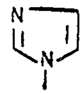 | 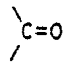 |
| 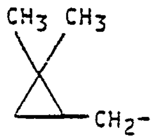 | 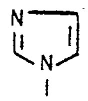 | 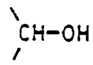 |
| 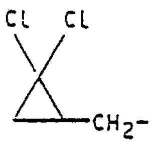 | 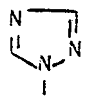 | 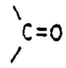 |
| 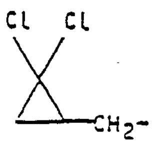 | 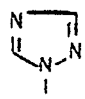 | 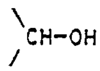 |
| 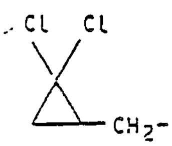 | 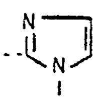 | 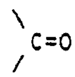 |
| 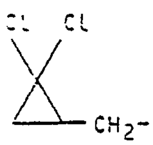 | 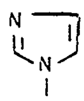 | 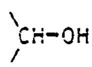 |
| 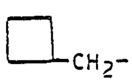 | 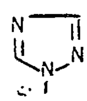 | 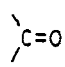 |
| 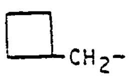 | 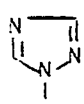 | 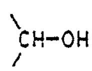 |
| 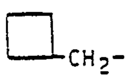 | 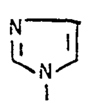 | 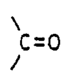 |
| 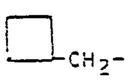 | 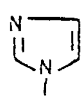 | 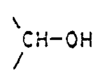 |

(Fortsetzung)

| R | Az | A |
|---|---|---|
| 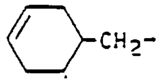 | 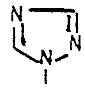 | C=O |
| 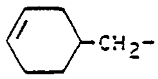 | 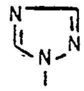 | CH-OH |
| 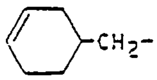 | 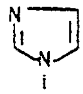 | C=O |
| 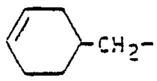 | 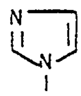 | CH-OH |
| 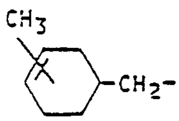 | 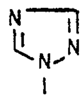 | C=O |
| 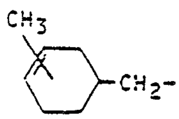 | 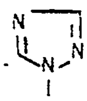 | CH-OH |
| 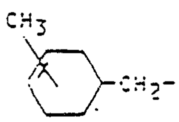 | 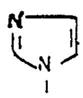 | C=O |
| 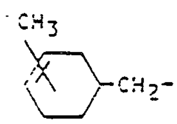 | 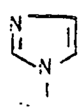 | CH-OH |
| 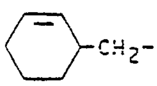 | 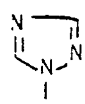 | C=O |
| 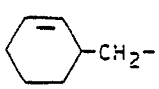 | 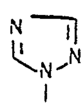 | CH-OH |

(Fortsetzung)

| R | Az | A |
|---|---|---|
| 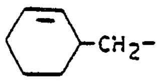 | 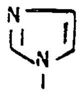 | C=O |
| 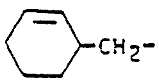 | 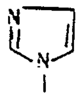 | CH-OH |
| 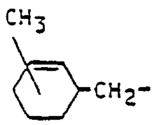 | 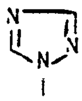 | C=O |
| 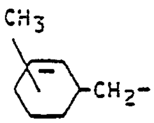 | 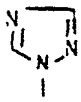 | CH-OH |
| 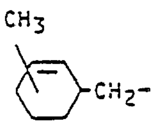 | 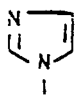 | C=O |
| 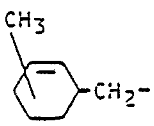 | 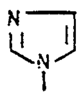 | CH-OH |
| 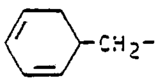 | 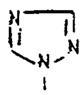 | C=O |
| 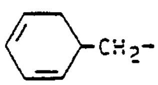 | 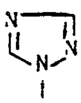 | CH-OH |
| 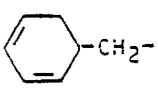 | 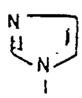 | C=O |
| 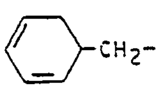 | 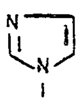 | CH-OH |

(Fortsetzung)

| R | Az | A |
|---|---|---|
| 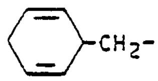 | 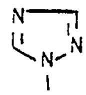 | >C=O |
| 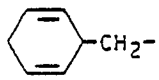 | 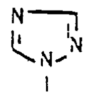 | >CH-OH |
| 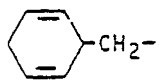 | 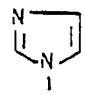 | >C=O |
| 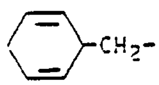 | 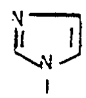 | >CH-OH |
| 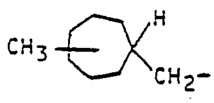 | 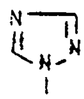 | >C=O |
| 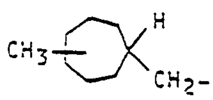 | 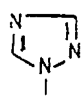 | >CH-OH |
| 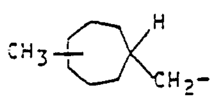 | 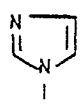 | >C=O |
| 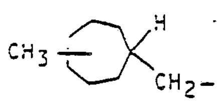 | 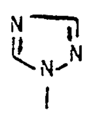 | >CH-OH |
| 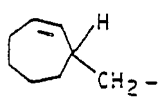 | 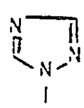 | >C=O |
| 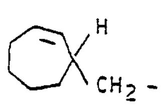 | 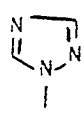 | >CH-OH |
| 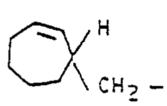 | 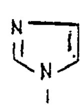 | >C=O |

(Fortsetzung)

| R | Az | A |
|---|---|---|
| 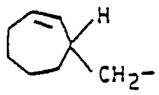 | 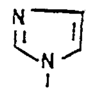 | CH-OH |
| 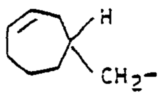 | 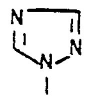 | C=O |
| 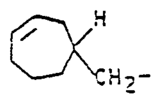 | 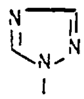 | CH-OH |
| 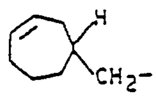 | 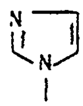 | C=O |
| 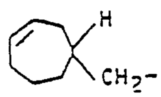 | 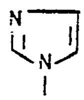 | CH-OH |
| 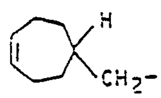 | 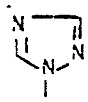 | C=O |
| 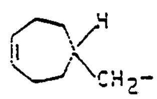 | 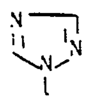 | CH-OH |
| 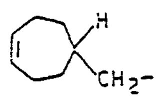 | 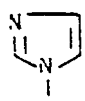 | C=O |
| 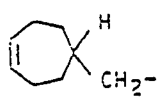 | 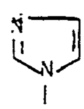 | CH-OH |
| 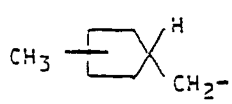 | 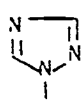 | C=O |
| 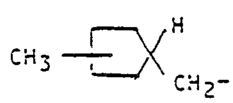 | 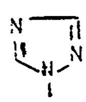 | CH-OH |
| 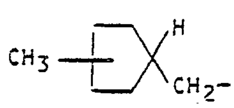 | 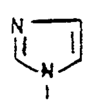 | C=O |
| 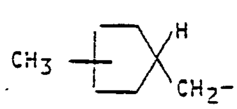 | 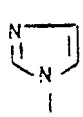 | CH-OH |

Verwendet man beispielsweise 3,3-Dimethyl-1-(1,2,4-triazol-1-yl)-butan-2-on und Brommethylcyclohexan als Ausgangsstoffe, so kann der Reaktionsablauf des erfindungsgemäßen Verfahrens durch das folgende Formelschema wiedergegeben werden :

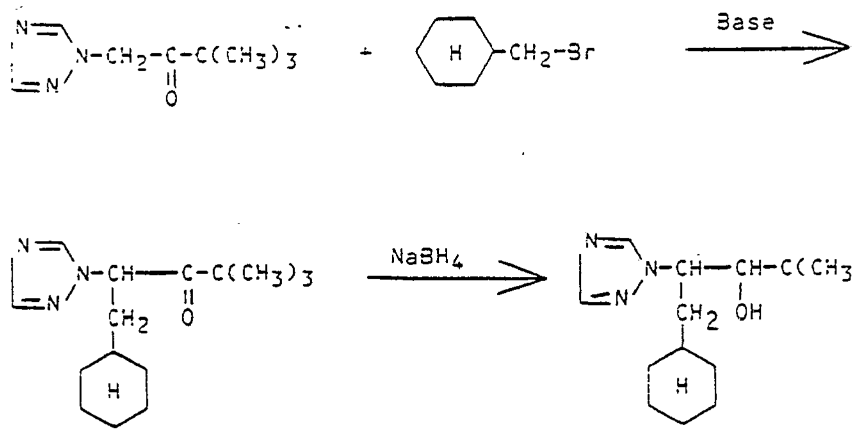


Die für die Durchführung des erfindungsgemäßen Verfahrens als Ausgangsstoffe benötigten Azolylmethyl-t-butyl-ketone sind durch die allgemeine Formel (II) allgemein definiert. In dieser Formel besitzt Az die in der Erfindungsdefinition angegebene Bedeutung.

Die Azolylmethyl-t-butyl-ketone der allgemeinen Formel (II) sind bekannt (vgl. DE-A 2 431 407, DE-A 2 906 061 und können in beschriebener Weise erhalten werden, wenn man Halogenketone wie z. B. Chlor- oder Brom-pinakol in Gegenwart eines Säurebindemittels mit Imidazol oder 1,2,4-Triazol umsetzt, vorzugsweise in Gegenwart von Calciumcarbonat oder Triethylamin in einem polaren Lösungsmittel (z. B. Acetonitril) im Temperaturbereich zwischen 60 und 120 °C.

Dis außerdem für das erfindungsgemäße Verfahren als Ausgangsstoffe zu verwendenden Alkylierungsmittel sind durch die allgemeine Formel (III) allgemein definiert. In dieser Formel steht R für diejenigen Reste, die bereits im Zusammenhang mit der Beschriebung der erfindungsgemäßen Stoffe der allgemeinen Formel (I) für diesen Substituenten genannt wurden. Z steht vorzugsweise für eine elektronenanziehende Abgangsgruppierung, wie biespielsweise für Halogen, wie Chlor, Brom oder Iod, für p-Methylphenylsulfonyloxy, für die Gruppierung $—O—SO_2—OR'$ oder $\overset{\oplus}{N}(R')_3$, wobei R' für Alkyl mit bis zu 4 Kohlenstoffatomen, Phenyl oder Tolyl steht.

Die Alkylierungsmittel der allgemeinen Formel (III) sind allgemein bekannte Verbindungen der organischen Chemie.

Für die erfindungsgemäße Umsetzung kommen als Verdünnungsmittel inerte organische Lösungsmittel infrage. Hierzu gehören vorzugsweise aromatische Kohlenwasserstoffe, wie Benzol, Toluol oder Xylol ; halogenierte Kohlenwasserstoffe, wie Methylenchlorid, Tetrachlorkohlenstoff, Chloroform oder Chlorbenzol ; Ester, wie Essigester ; Formamide, wie Dimethylformamid ; sowie Dimethylsulfoxid.

Die erfindungsgemäße Umsetzung wird in Gegenwart einer Base durchgeführt. Hierbei können alle üblichen organischen und insbesondere anorganischen Basen eingesetzt werden, wie vorzugsweise Alkalihydroxide oder Alkalicarbonate, beispielsweise seien Natrium- und Kaliumhydroxid genannt.

Die Reaktionstemperaturen können bei der Durchführung des erfindungsgemäßen Verfahrens in einem größeren Bereich variiert werden. Im allgemeinen arbeitet man zwischen 0 und 120 °C, vorzugsweise zwischen 20 und 100 °C.

Bei der Durchführung des erfindungsgemäßen Verfahrens, setzt man vorzugsweise auf 1 Mol Triazolylmethyl-tert.-butyl-keton der allgemeinen Formel (II) 1 bis 1,2 Mol Alkylierungsmittel der allgemeinen Formel (III) ein. Die Isolierung der Keto-Verbindungen der weiter unten angegebenen allgemeinen Formel (Ia) erfolgt in allgemein üblicher Art und Weise.

Die erfindungsgemäße Umsetzung kann auch in einen Zweiphasensystem, wie beispielsweise wässrige Natron- oder Kalilauge/Toluol oder Methylenchlorid, gegebenenfalls unter Zusatz von 0,1 bis 1 Mol eines Phasen-Transfer-Katalysators, wie beispielsweise Ammonium- oder Phosphoniumverbindungen, beispielsweise seien Benzyldodecyldimethyl-ammoniumchlorid und Triethyl-benzylammoniumchlorid genannt, durchgeführt werden.

Die gegebenenfalls erforderliche Reduktion der Keto-Verbindungen der allgemeinen Formel (Ia)

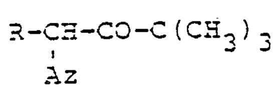

(Ia)

zu den Verbindungen der allgemeinen Formel (Ib)

$$R-\underset{Az}{\underset{|}{CH}}-\underset{OH}{\underset{|}{CH}}-C(CH_3)_3 \qquad (Ib)$$

wobei in obigen allgemeinen Formeln (Ia) und (Ib) die Symbole R und Az die in allgemeiner Formel (I) angegebene Bedeutung besitzen, erfolgt in üblicher Weise, wie z. B. durch Umsetzung mit komplexen Hydriden, gegebenenfalls in Gegenwart eines Verdünnungsmittels ; oder durch Umsetzung mit Aluminium-isopropylat in Gegenwart eines Verdünnungsmittels ; oder durch Umsetzung mit Wasserstoff in Gegenwart eines Katalysators und gegebenenfalls in Gegenwart eines Verdünnungsmittels.

Arbeitet man mit komplexen Hydriden, so kommen als Verdünnungsmittel für die Reduktions-Reaktion polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30 °C, vorzugsweise bei 0 bis 20 °C durchgeführt. Hierzu setzt man auf 1 Mol des Ketones der allgemeinen Formel (Ia) etwa 1 Mol eines komplexen Hydrids, wie Natriumborhydrid oder Lithiumalanat, ein. Zur Isolierung der reduzierten Verbindungen der allgemeinen Formel (Ib) wird der Rückstand in verdünnter Salzsäure aufgenommen, anschließend alkalisch eingestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die oben erwähnte Reduktion bevorzugt Alkohole, wie Isopropanol, oder inerte Kohlenwasserstoffe, wie Benzol, infrage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der allgemeinen Formel (Ia) etwa 1 bis 2 Mol Aluminiumisopropylat ein. Zur Isolierung der reduzierten Verbindungen der allgemeinen Formel (Ib) wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Arbeitet man mit Wasserstoff, so kommen als Verdünnungsmittel für die durchzuführende Reduktion polare organische Lösungsmittel infrage. Hierzu gehören vorzugsweise Alkohole, wie Methanol und Ethanol ; sowie Nitrile, wie Acetonitril. Die Umsetzung wird in Gegenwart eines Katalysators vorgenommen. Vorzugsweise werden Edelmetall-, Edelmetalloxid-bzw. Edelmetallhydroxid-Katalysatoren oder sogenannte « Raney-Katalysatoren » verwendet, insbesondere Platin, Platinoxid und Nickel. Die Reaktionstemperaturen können in einem größeren Bereich variiert werden. Im allgemeinen arbeitet man zwischen 20 und 50 °C. Die Reaktion kann bei Normaldruck, aber auch bei erhöhtem Druck, beispielsweise 1 bis 2 atü, durchgeführt werden. Zur Durchführung der Reaktion setzt man auf 1 Mol der Verbindung der allgemeinen Formel (Ia) etwa 1 Mol Wasserstoff und 0,1 Mol Katalysator ein. Zur Isolierung der reduzierten Verbindungen der allgemeinen Formel (Ib) wird vom Katalysator abfiltriert und vom Lösungsmittel im Vakuum befreit. Die weitere Aufarbeitung erfolgt in üblicher Weise.

Die erfindungsgemäß herstellbaren Verbindungen der allgemeinen Formel (I) können in Säureadditionssalze bzw. Metallsalz-Komplexe überführt werden.

Zur Herstellung von pflanzenverträglichen Säureadditionssalzen der Verbindungen der allgemeinen Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Furmarsäure, Weinsäure, Citronensäure, Salicylsäure, Sorbinsäure, Milchsäure sowie Sulfonsäuren, wie z. B. p-Toluolsulfonsäure und 1,5-Naphthalindisulfonsäure. Die Säureadditions-Salze der Verbindungen der allgemeinen Formel (I) können in einfacher Weise nach üblichen Salzbildungsmethoden, z. B. durch Lösen einer Verbindung der allgemeinen Formel (I) in einem geeigneten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol und Hinzufügen zur Verbindung der allgemeinen Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Rostpilzen, wie z. B. gegen den Erreger des Weizenbraunrostes (Puccinia recondita), gegen Echte Mehltaupilze, gegen weitere Krankheitserreger im Getreidebau, wie z. B. gegen den Erreger der Streifenkrankheit der Gerste (Drechslera graminea), gegen Cochliobolus-Arten wie Cochliobolus sativus, gegen Pyrenophora-Arten wie Pyrenophora teres, gegen verschiedene Krankheitserreger im Reisanbau und gegen den Grauschimmel (Botrytis cinerea) eingesetzt werden.

Daneben besitzen die erfindungsgemäßen Wirkstoffe auch eine ausgeprägte bakterizide Wirkung. Zu erwähnen ist ferner bei entsprechend höheren Aufwandmengen eine herbizide Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, Lösliche Pulver, Granulate, Aerosole, Suspensions-Emulsions-Konzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hiltslösungsmittel verwendet werden. Als flüssige Lösungsmittel kommen im wesentlichen infrage : Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen infrage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen infrage : z. B. gebrochene und fraktionierte natürliche Gesteine wie Calcit, Mamor, Bims, Sepiolith, Dolomit sowie synthetische Granulate aus anorganischen und organischen Mehlen sowie Granulate aus organischem material, wie Sägemehl, Kokosnußschalen, Maiskolben und Tabakstengeln ; als Emulgier- und/oder schaumerzeugende Mittel kommen infrage : z. B. nichtionogene und anionische Emulgatoren, wie Polyoxyethylen-Fettsäure-ester, Polyoxyethylen-Fettalkoholether, z. B. Alkylarylpolyglykol-ether, Alkylsulfonate, Alkylsulfate, Arylsulfonate sowie Eiweißhydrolysate ; als Dispergiermittel kommen infrage : z. B. Lignin-Sulfitablaugen und Methylcellulose.

Es können in den Formulierungen Haftmittel wie Carboxymethylcellulose, natürliche und synthetische pulverige, körnige oder Latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink, verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

### Beispiel 1

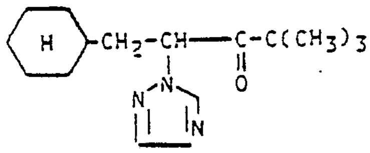

4,8 g Natriumhydrid (80 %-ige Suspension in Mineralöl, 0,16 Mol) werden in 50 ml absol. Dimethylformamid suspendiert, dazu tropft man bei 20 bis 25 °C unter Rühren 26,8 g (0,16 Mol) 3,3-Dimethyl-1-(1,2,4-triazol-1-yl)-butan-2-on in 50 ml absol. Dimethylformamid. Nach Beendigung der Wasserstoffentwicklung tropft man bei 20 bis 25 °C 30,8 g (0,17 Mol) Brommethylcyclohexan zu und erwärmt anschließend für 4 Stunden auf 80 °C. Nach dem Abkühlen der Reaktionsmischung gießt man diese vorsichtig in 1 l Wasser und extrahiert mehrmals mit Methylenchlorid. Die vereinigten organischen Extrakte werden über Natriumsulfat getrocknet und im Vakuum eingedampft. Durch Destillation des Rückstandes erhält man 29,5 g (70 % der Theorie) an 1-Cyclohexyl-4,4-dimethyl-2-(1,2,4-triazol-1-yl)-pentan-3-on vom Siedepunkt $Kp_{0,1}$/135 bis 140 °C als schwach gelb gefärbtes Oel.

### Beispiel 2

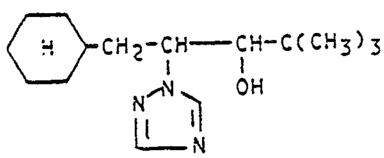

13,2 g (0,05 Mol) 1-Cyclohexyl-4,4-dimethyl-2-(1,2,4-triazol-1-yl)-pentan-3-on werden in einem Gemisch aus 100 ml Methanol und 20 ml Wasser gelöst, in mehreren Portionen mit insgesamt 2 g (0,05 Mol) Natriumborhydrid versetzt und über Nacht gerührt. Zur Aufarbeitung wird das Reaktionsgemisch mit verdünnter Salzsäure auf pH 5 gebracht und das Lösungsmittel im Vakuum abgedampft. Der Rückstand wird in Wasser aufgenommen und mehrfach mit Methylenchlorid extrahiert. Die vereinigten organischen Extrakte werden mit Natriumhydrogencarbonatlösung und Wasser gewaschen, über Natriumsulfat getrocknet und im Vakuum eingedampft. Der Rückstand wird mit Petrolether zur Kristallisation gebracht. Man erhält 10 g (75,2 % der Theorie) an 1-Cyclohexyl-4,4-dimethyl-2-(1,2,4-triazol-1-yl)-pentan-3-ol in Form farbloser Kristalle vom Schmelzpunkt 104 bis 107 °C.

In analoger Weise werden die folgenden Verbindungen der allgemeinen Formel (I) erhalten :

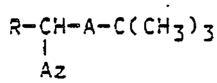

(I)

| Beisp.-Nr. | R | Az | A | physikalische Eigenschaften |
|---|---|---|---|---|
| 3 | 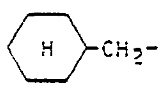 | 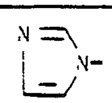 | 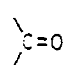 | $Kp_{0,1}$/ 120°C |
| 4 | 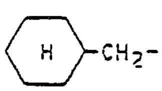 | 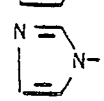 | 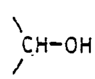 | Fp. 138°C |
| 5 | 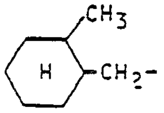 | 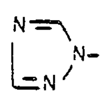 | 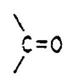 | $Kp_{0,1}$/150°C |
| 6 | 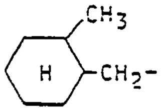 | 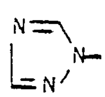 | 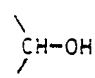 | Fp. 51°C |
| 7 | 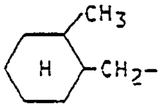 | 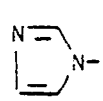 | 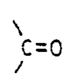 | $Kp_{0,2}$/160°C |

(Fortsetzung)

| Beisp.-Nr. | R | Az | A | physikalische Eigenschaften |
|---|---|---|---|---|
| 8 | 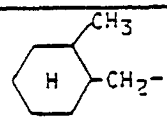 | 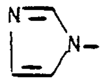 | >CH-OH | Fp. 78°C |
| 9 | 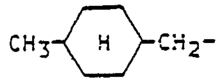 | 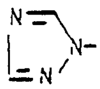 | >C=O | $Kp_{0,1}$/ 160°C |
| 10 | 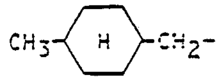 | 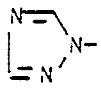 | >CH-OH | $n_D^{20}$ = 1,4978 |
| 11 | 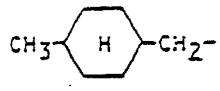 | 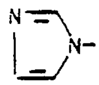 | >CH-OH | Oel |
| 12 | 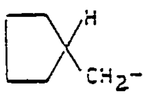 | 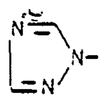 | >C=O | $Kp_{0,1}$/140°C |
| 13 | 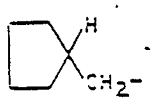 | 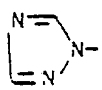 | >CH-OH | Fp. 47°C |
| 14 | 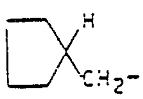 | 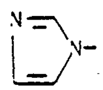 | >C=O | Fp. 98°C |
| 15 | 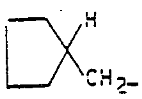 | 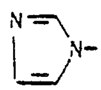 | >CH-OH | Fp. 127°C |
| 16 | 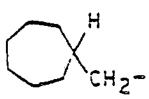 | 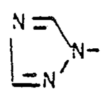 | >C=O | $n_D^{20}$ = 1,5107 |
| 17 | 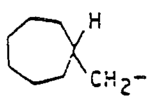 | 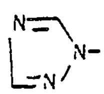 | >CH-OH | Fp. 98-104°C |
| 18 | 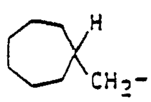 | 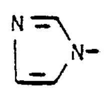 | >C=O | $n_D^{20}$ = 1,5064 |
| 19 | 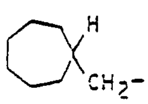 | 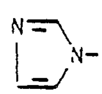 | >CH-OH | Oel |

## Anwendungsbeispiele

Bei der Prüfung auf fungizide Wirksamkeit werden in den folgenden Beispielen die nachstehend angegebenen Verbindungen als Vergleichssubstanzen eingesetzt :

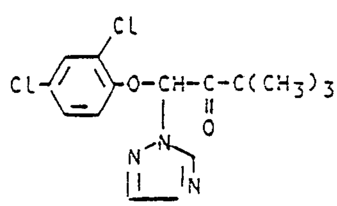

(A)

1-(2,4-Dichlorphenoxy)-3,3-dimethyl-1-(1,2,4-triazol-1-yl)-butan-2-on

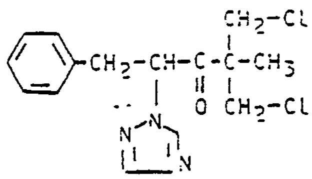

(B)

4,4-Bis-(chlormethyl)-1-phenyl-2-(1,2,4-triazol-1-yl)-pentan-3-on

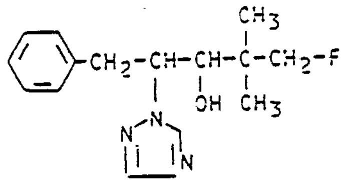

(C)

2,2-Dimethyl-1-fluor-5-phenyl-4-(1,2,4-triazol-1-yl)-pentan-3-ol

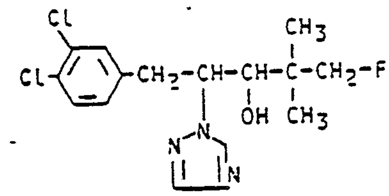

(D)

5-(3,4-Dichlorphenyl)-2,2-dimethyl-1-fluor-4-(1,2,4-triazol-1-yl)-pentan-3-ol.

Beispiel A

Botrytis-Test (Bohne)/protektiv

Lösungsmittel : 4,7 Gewichtsteile Aceton
Emulgator : 0,3 Gewichtsteile Alkylarylpolyglycolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 4 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigt in diesem Test z. B. die Verbindung gemäß Herstellungsbeispiel (2).

Beispiel B

Puccinia-Test (Weisen)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20 °C und 100 % rel. Luftfeuchtigeit in einer Inkubationskabine.

Die Pflanzen werden in einem Gewächshaus bei einer Temperatur von ca. 20 °C und einer relativen Luftfeuchtigkeit von ca. 30 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß Herstellungsbeispiele (2) u. (4).

Beispiel C

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde unk kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigt bei diesem Test z. B. die Verbindung gemäß Herstellungsbeispiel (2).

**Patentansprüche**

1. Substituierte Azolylalkyl-t-butyl-ketone und -carbinole der allgemeinen Formel (I)

$$R-\underset{\underset{Az}{|}}{CH}-A-C(CH_3)_3 \qquad (I)$$

in welcher

Az für Imidazol-1-yl oder 1,2,4-Triazol-1-yl steht,

A für die Carbonylgruppe oder die Hydroxymethylengruppe steht und

R für gegebenenfalls ein- bis fünffach durch Halogen, Alkyl mit bis zu 4 Kohlenstoffatomen und/oder Alkoxy mit bis zu 4 Kohlenstoffatomen gleich oder verschieden substituiertes Cycloalkylalkyl und Cycloalkenylalkyl mit jeweils 3 bis 9 Kohlenstoffatomen im Cycloalkyl- bzw. Cycloalkenylteil und bis zu 4 Kohlenstoffatomen im jeweiligen Alkyl steht,

sowie deren pflanzenverträgliche Säureadditions-Salze und Metallsalz-Komplexe.

2. Verbindungen der allgemeinen Formel (I) in Anspruch 1, wobei

Az und A die in Anspruch 1 angegebene Bedeutung besitzen und

R für gegebenenfalls ein- bis dreifach, gleich oder verschieden durch Methyl, Methoxy, Ethyl oder Chlor substituiertes Cyclopropylmethyl, Cyclobutylmethyl, Cyclopentylmethyl, Cyclohexylmethyl, Cyclohexenylmethyl, Cyclohexadienylmethyl, Cycloheptylmethyl oder Cycloheptenylmethyl steht.

3. Verfahren zur Herstellung von substituierten Azolyl-alkyl-t-butyl-ketonen und -carbinolen der allgemeinen Formel (I)

$$R-\underset{\underset{Az}{|}}{CH}-A-C(CH_3)_3 \qquad (I)$$

in welcher

Az für Imidazol-1-yl oder 1,2,4-Triazol-1-yl steht,

A für die Carbonylgruppe oder Hydroxymethylengruppe steht und

R für gegebenenfalls ein- bis fünffach durch Halogen, Alkyl mit bis zu 4 Kohlenstoffatomen oder Alkoxy mit bis zu 4 Kohlenstoffatomen gleich oder verschieden substituiertes Cycloalkylalkyl und Cycloalkenylalkyl mit jeweils 3 bis 9 Kohlenstoffatomen im Cycloalkyl- bzw. Cycloalkenylteil und bis zu 4 Kohlenstoffatomen im jeweiligen Alkyl steht,

sowie deren pflanzenverträglichen Säureadditions-Salzen und Metallsalz-Komplexen, dadurch gekennzeichnet, daß man Azolylmethyl-t-butyl-ketone der allgemeinen Formel (II)

$$Az-CH_2-\underset{\underset{O}{\|}}{C}-C(CH_3)_3 \qquad (II)$$

in welcher Az die oben angegebene Bedeutung hat, mit einem Alkylierungsmittel der allgemeinen Formel (III)

$$R-Z \qquad (III)$$

in welcher

R die oben angegebene Bedeutung hat und

Z für eine elektronenanziehende Abgangsgruppierung steht,

in Gegenwart einer Base und in Gegenwart eines organischen Verdünnungsmittels, oder in einem wässrigorganischen Zweiphasensystem in Gegenwart eines Phasentransferkatalysators, umsetzt und gegebenenfalls die dabei erhaltenen Keto-Verbindungen nach bekannten Methoden zu den entsprechenden Carbinolen reduziert, und gegebenenfalls an die erhaltenen Verbindungen der allgemeinen Formel (I) eine pflanzenverträgliche Säure oder ein pflanzenverträgliches Metallsalz addiert.

4. Pflanzenschutzmittel, gekennzeichnet, durch einen Gehalt an mindestens einem substituierten Azolyl-alkyl-t-butyl-keton oder -carbinol der allgemeinen Formel (I) in Anspruch 1 bzw. einem pflanzenverträglichen Säureadditions-Salz oder Metallsalz-Komplex eines Stoffes der allgemeinen Formel (I).

5. Verfahren zur Bekämpfung von Pilzen und Bakterien, dadurch gekennzeichnet, daß man substituierte Azolylalkyl-t-butyl-ketone und -carbinole der allgemeinen Formel (I) in Anspruch 1 bzw. deren pflanzenverträgliche Säureadditions-Salze oder Metallsalz-Komplexe auf Pilze oder Bakterien oder ihren Lebensraum einwirken läßt.

6. Verwendung von substituierten Azolylalkyl-t-butyl-ketonen und -carbinolen der allgemeinen Formel (I) in Anspruch 1 bzw. von deren pflanzenverträglichen Säureadditions-Salzen oder Metallsalz-Komplexen als Pflanzenschutzmittel.

7. Verwendung von substituierten Azolylalkyl-t-butyl-ketonen oder -carbinolen der allgemeinen Formel (I) in Anspruch 1 bzw. von deren pflanzenverträglichen Säureadditions-Salzen oder Metallsalz-Komplexen zur Bekämpfung von Pilzen und Bakterien.

8. Verfahren zur Herstellung von Pflanzenschutzmitteln, dadurch gekennzeichnet, daß man substituierte Azolylalkyl-t-butyl-ketone oder -carbinole der allgemeinen Formel (I) in Anspruch 1 bzw. deren pflanzenverträgliche Säureadditions-Salze oder Metallsalz-Komplexe mit Streckmitteln und/oder oberflächenaktiven Mitteln vermischt.

**Claims**

1. Substituted azolylalkyl-t-butyl-ketones and -carbinols of the general formula (I)

$$R-\underset{Az}{CH}-A-C(CH_3)_3 \qquad (I)$$

in which

Az represents imidazol-1-yl or 1,2,4-triazol-1-yl,

A represents the carbonyl group or the hydroxymethylene group and

R represents cycloalkylalkyl and cycloalkenylalkyl, each of which has 3 to 9 carbon atoms in the cycloalkyl or cycloalkenyl part and up to 4 carbon atoms in the respective alkyl and is optionally mono- to pentasubstituted by identical or different substituents from amongst halogen, alkyl with up to 4 carbon atoms and/or alkoxy with up to 4 carbon atoms,

and their plant-tolerated acid addition salts and metal salt complexes.

2. Compounds of the general formula (I) in Claim 1, wherein

Az and A have the meaning given in Claim 1 and

R represents cyclopropylmethyl, cyclobutylmethyl, cyclopentylmethyl, cyclohexylmethyl, cyclohexenylmethyl, cyclohexadienylmethyl, cycloheptylmethyl or cycloheptenylmethyl, optionally mono- to trisubstituted by identical or different substituents from amongst methyl, methoxy, ethyl or chlorine.

3. Process for the preparation of substituted azolylalkyl-t-butyl-ketones and -carbinols of the general formula (I)

$$R-\underset{Az}{CH}-A-C(CH_3)_3 \qquad (I)$$

in which

Az represents imidazol-1-yl or 1,2,4-triazol-1-yl,

A represents the carbonyl group or the hydroxymethylene group and

R represents cycloalkylalkyl and cycloalkenylalkyl, each of which has 3 to 9 carbon atoms in the cycloalkyl or cycloalkenyl part and up to 4 carbon atoms in the respective alkyl and is optionally mono- to pentasubstituted by identical or different substituents from amongst halogen, alkyl with up to 4 carbon atoms or alkoxy with up to 4 carbon atoms,

and their plant-tolerated acid addition salts and metal salt complexes, characterised in that azolylmethyl-t-butyl-ketones of the general formula (II)

$$Az-CH_2-\underset{\overset{\|}{O}}{C}-C(CH_3)_3 \qquad (II)$$

in which Az has the meaning given above, are reacted with an alkylating agent of the general formula (III)

$$R—Z \qquad (III)$$

in which

R has the meaning given above and

Z represents an electron-attracting leaving grouping,

in the presence of a base and in the presence of an organic diluent, or in an aqueous-organic two-phase system in the presence of a phase-transfer catalyst and, if required, the resulting keto compounds are reduced by known methods to the corresponding carbinols and, if required, a plant-tolerated acid or a plant-tolerated metal salt is added on to the compounds of the general formula (I) obtained.

4. Plant-protecting agents, characterised in that they contain at least one substituted azolylalkyl-t-butyl-ketone or -carbinol of the general formula (I) in Claim 1 or a plant-tolerated acid addition salt or metal salt complex of a compound of the general formula (I).

5. Process for combating fungi and bacteria, characterised in that substituted azolylalkyl-t-butyl-ketones and -carbinols of the general formula (I) in Claims 1 or their plant-tolerated acid addition salts or metal salt complexes are allowed to act on fungi or bacteria or their habitat.

6. Use of substituted azolylalkyl-t-butyl-ketones and -carbinols of the general formula (I) in Claim 1 or their plant-tolerated acid addition salts or metal salt complexes as plant-protection agents.

7. Use of substituted azolylalkyl-t-butyl-ketones or -carbinols of the general formula (I) in Claim 1 or their plant-tolerated acid addition salts or metal salt complexes for combating fungi and bacteria.

8. Process for the preparation of plant protection agents, characterised in that substituted azolylalkyl-t-butyl-ketones or -carbinols of the general formula (I) in Claim 1 or their plant-tolerated acid addition salts or metal salt complexes are mixed with extenders and/or surface-active agents.

**Revendications**

1. Azolylalkyl-tert.-butyl-cétones et -carbinols substitués de formule générale I

$$R-\underset{\underset{Az}{|}}{CH}-A-C(CH_3)_3 \qquad (I)$$

dans laquelle

Az represente un reste imidazole-1-yle ou 1,2,4-triazole-1-yle,

A représente un groupe carbonyle ou un groupe hydroxyméthylène, et

R représente un groupe cycloalkylalkyle ou cycloalcénylalkyle portant éventuellement 1 à 5 substituants identiques ou différents choisis parmi les atomes d'halogènes, les groupes alkyle contenant jusqu'à 4 atomes de carbone et/ou alcoxy contenant jusqu'à 4 atomes de carbone, avec chacun 3 à 9 atomes de carbone dans la partie cycloalkyle ou cycloalcényle et jusqu'à 4 atomes de carbone dans la partie alkyle,

et leurs sels formés par addition avec des acides et complexes de sels métalliques tolérés par les végétaux.

2. Composés de formule générale I de la revendication 1, dans laquelle

Az et A ont les significations indiquées dans la revendication 1, et

R représente un groupe cyclopropylméthyle, cyclobutylméthyle, cyclopentylméthyle, cyclohexylméthyle, cyclohexénylméthyle, cyclohexadiénylméthyle, cycloheptylméthyle ou cyclohepténylméthyle portant éventuellement 1 à 3 substituants identiques ou différents choisis parmi les groupes méthyle, méthoxy, éthyle ou les atomes de chlore.

3. Procédé de préparation des azolylalkyl-tert.-butyl-cétones et -carbinols de formule générale I

$$R-\underset{\underset{Az}{|}}{CH}-A-C(CH_3)_3 \qquad (I)$$

dans laquelle

AZ représente un groupe imidazole-1-yle ou 1,2,4-triazole-1-yle,

A représente un groupe carbonyle ou un groupe hydroxyméthylène, et

R représente un groupe cycloalkylalkyle ou cycloalcénylalkyle portant éventuellement 1 à 5 substituants identiques ou différents choisis parmi les atomes d'halogènes, les groupes alkyle contenant

jusqu'à 4 atomes de carbone ou alcoxy contenant jusqu'à 4 atomes de carbone, avec chacun 3 à 9 atomes de carbone dans la partie cycloalkyle ou cycloalcényle et jusqu'à 4 atomes de carbone dans la partie alkyle,

et de leurs sels formés par addition avec des acides et complexes de sels métalliques tolérés par les végétaux, caractérisé en ce que l'on fait réagir des azolylméthyl-tert.-butyl-cétones de formule générale II

$$Az-CH_2-\underset{\underset{O}{\parallel}}{C}-C(CH_3)_3 \qquad (II)$$

dans laquelle Az a les significations indiquées ci-dessus, avec un agent alkylant de formule générale III

$$R—Z \qquad (III)$$

dans laquelle

R a les significations indiquées ci-dessus, et

Z représente un groupement éliminable accepteur d'électrons,

en présence d'une base et en présence d'un diluant organique, ou dans un système à deux phases hydro-organique en présence d'un catalyseur à transfert de phases, et le cas échéant on réduit les composés cétoniques obtenus, selon des procédés connus, en les carbinols correspondants et le cas échéant, sur les composés de formule générale I ainsi obtenus, on fixe par addition un acide toléré par les végétaux ou un sel métallique toléré par les végétaux.

4. Produit phytosanitaire caractérisé en ce qu'il contient au moins une azolylalkyl-tert.-butyl-cétone ou un azolylalkyl-tert.-butyl-carbinol substitués de formule générale I de la revendication 1, ou un sel formé par addition avec un acide toléré par les végétaux ou complexe de sel métallique toléré par les végétaux d'une substance de formule générale I.

5. Procédé pour combattre les mycètes et les bactéries, caractérisé en ce que l'on fait agir sur les mycètes ou les bactéries ou sur leur habitat des azolylalkyl-tert.-butyl-cétones et -carbinols substitués de formule générale I de la revendication 1 ou leurs sels formés par addition avec des acides ou complexes de sels métalliques tolérés par les végétaux.

6. Utilisation des azolylalkyl-tert.-butyl-cétones et -carbinols substitués de formule générale I de la revendication 1 ou de leurs sels formés par addition avec des acides ou complexes de sels métalliques tolérés par les végétaux en tant que produits phytosanitaires.

7. Utilisation des azolylalkyl-tert.-butyl-cétones et -carbinols substitués de formule générale I de la revendication 1 ou de leurs sels formés par addition avec des acides ou complexes de sels métalliques tolérés par les végétaux pour combattre les mycètes et les bactéries.

8. Procédé de préparation de produits phytosanitaires, caractérisé en ce que l'on mélange les azolyalkyl-tert.-butyl-cétones ou -carbinols substitués de formule générale I de la revendication 1 ou leurs sels formés par addition avec des acides ou complexes de sels métalliques tolérés par les végétaux avec des diluants et/ou des agents tensioactifs.